# FASCICULE DE BREVET EUROPEEN

(11) **EP 3 442 333 B1**
(45) Date de publication et mention de la délivrance du brevet: **07.04.2021**
(21) Numéro de dépôt: 17720557.2
(22) Date de dépôt: 07.04.2017
(51) Int. Cl.: A01N 27/00, A01N 61/02, A01N 25/02, A01P 3/00

(54) **COMPOSITION D'HUILE PHYTOSANITAIRE PARAFFINIQUE**
PARAFFINISCHE PHYTOSANITÄRE ÖLZUSAMMENSETZUNG
PARAFFINIC PHYTOSANITARY OIL COMPOSITION

(30) Priorité: 15.04.2016 FR 1653333
(43) Date de publication de la demande: 20.02.2019
(73) Titulaire: Total Marketing Services, 92800 Puteaux (FR)
(72) Inventeur: QUILLET, Serge, 78220 VIROFLAY (FR); BUREAU, Eric, 78125 ST-HILARION (FR); CUKIERMAN, Serge, 92200 Neuilly Sur Seine (FR); PLANCQ, Louis, 92300 LEVALLOIS-PERRET (FR)
(74) Mandataire: Corizzi, Valérie
(86) Numéro de dépôt international: PCT/FR2017/050837
(87) Numéro de publication internationale: WO 2017/178738

(56) Documents cités:
- EP-A2- 0 255 871
- EP-A2- 0 255 871
- WO-A1-2013/092977
- WO-A1-2013/092977
- WO-A2-2010/103245
- FR-A1- 2 953 369
- FR-A1- 2 953 369
- FR-A1- 2 999 190
- FR-A1- 2 999 190

## Description

### DOMAINE DE L'INVENTION

L'invention concerne une composition à base d'huile phytosanitaire de type coupe hydrocarbonée paraffinique pour le traitement des maladies cryptogamiques des plantes ainsi que le procédé de traitement et l'utilisation de cette coupe hydrocarbonée et de cette composition à titre préventif et curatif, notamment sur les premiers stades des maladies cryptogamiques des plantes.

### CONTEXTE TECHNIQUE DE L'INVENTION

Une maladie cryptogamique, ou maladie fongique, est une maladie causée à une plante par un champignon ou un autre organisme filamenteux parasite. Parmi ces maladies on peut notamment citer la cercosporiose noire ou encore l'oïdium. Les spores des champignons, le plus souvent transportés par le vent, se déposent sur les plantes, germent et pénètrent à l'intérieur des tissus de la plante. Le champignon passe par les orifices naturels (stomates, lenticelles) ou pénètre par des blessures (notamment celles provoquées par des insectes ou par des tailles de branches), ou encore en traversant la cuticule de la plante.

La cercosporiose noire ou maladie des raies noires du bananier, causée par le champignon ascomycète *Mycosphaerella fijiensis,* et la cercosporiose jaune, causée par le champignon ascomycète *Mycosphaerella musicola,* sont les maladies foliaires les plus destructrices de la culture bananière. Les attaques réduisent la photosynthèse, et, si le nombre de feuilles fonctionnelles est insuffisant entre la floraison et la récolte, le régime mûrit sur pied prématurément, privant ainsi le producteur de sa commercialisation.

Dans le cas de la cercosporiose, le contrôle de la maladie peut être effectué en utilisant divers types de fongicides. Les fongicides de contact agissent lorsqu'ils sont en contact avec le champignon. Les fongicides systémiques doivent être absorbés par la plante pour agir. L'usage de fongicides permet de lutter contre les cercosporioses dans les plantations commerciales, mais leurs effets sur l'environnement sont préoccupants. Bien qu'il soit possible de réduire sensiblement le nombre de traitements si ceux-ci sont pratiqués dans le cadre d'une lutte raisonnée, des souches de *Mycosphaerella fijiensis* et de *M*. *musicola* ont développé une résistance à de nombreux produits systémiques dans toutes les régions de production du globe (soit en Amérique Latine, dans les Caraïbes, en Afrique et en Asie).

Il est connu d'utiliser des compositions phytosanitaires pour assurer la protection des cultures, ces compositions pouvant remplir le rôle de fongicide ou encore d'insecticide. Elles sont généralement pulvérisées sur les cultures sous forme de solutions dans l'eau ou d'émulsions eau/huile ou huile/eau, en présence d'additifs tensioactifs. L'huile présente elle-même un effet fongistatique bien connu participant au ralentissement du développement de la maladie.

Depuis longtemps, des huiles d'origine pétrolière émulsionnées dans l'eau sont pulvérisées sur les cultures dans un but insecticide. Convenablement préparées, de telles huiles permettent d'affecter les champignons et parasites tout en n'ayant aucun effet négatif sur le développement des plantes. Parmi les huiles raffinées conventionnelles, obtenues à partir de pétrole brut et/ou des distillats issus du raffinage, par extraction au solvant ou par hydrotraitement d'huiles de base, l'industriel choisira celles qui seront les moins toxiques mais les plus efficaces, par exemple à l'encontre des insectes et/ou des attaques fongiques. Il a été ainsi constaté que plus l'huile fabriquée est paraffinique, c'est-à-dire comprend des composés de chaîne carbonée saturée, linéaire ou branchée, plus celle-ci est efficace à l'encontre des insectes et moins elle est phytotoxique pour les plantes.

Cependant même dans des publications très larges, comme Spray Oils Beyond 2000 : Proceedings of a conference held from 1999 in Sydney, Published by the University of Western Sydney, Australia*,* abordant l'incidence de la nature de l'huile (pureté, nombre de carbones moyen, intervalle de coupe, etc), ou encore dans les publications du Centre de coopération Internationale en Recherche Agronomique pour le Développement (CIRAD), ayant travaillé sur cette thématique depuis les années 1950, en particulier sur les cultures tropicales, aucune information n'est retrouvée sur une différence de performance associée aux caractéristiques intrinsèques de l'huile paraffinique. Seule l'importance d'une teneur élevée en paraffines, sans distinction faite sur ces paraffines, est largement décrite.

Les produits rencontrés sur le marché du traitement de la Cercosporiose ont en général une viscosité cinématique à 40°C allant de 10 à 20 cSt et des propriétés à froid améliorées par un procédé de déparaffinage au solvant ou déparaffinage catalytique. D'autre part et jusqu'à présent, une relation entre viscosité des compositions paraffiniques phytosanitaires et efficacité fongistatique était communément faite par les acteurs de la profession. En effet, il est considéré aujourd'hui, qu'une viscosité élevée permet à l'huile appliquée de rester plus longtemps sur les feuilles à traiter et ainsi d'être plus efficace. Toutefois avec une viscosité élevée, comme celle de la majorité des huiles généralement utilisées dans le domaine, la composition d'huile utilisée reste de façon prolongée sur les feuilles des plantes traitées et peut donc affecter le métabolisme des plantes en réduisant la capacité photosynthétique de celles-ci.

D'autre part, dans Spray Oils Beyond 2000 : Proceedings of a conference held from 1999 in Sydney, Published by the University of Western Sydney, Australia*,* il est indiqué que les chaines carbonées comprenant plus de 26 atomes de carbone peuvent conduire à une phytotoxicité chronique.

Divers procédés de traitement fongicide des cultures sont décrits dans la littérature. La demande WO03/073858 décrit un procédé de lutte contre les maladies des végétaux par inhibition des enzymes extracellulaires des microorganismes contaminants. Cette demande démontre l'efficacité du bore, sous forme d'acide borique, dans l'inhibition des enzymes dégradatives extracellulaires. Cependant, de telles solutions ou suspensions aqueuses ne conviennent pas pour l'épandage aérien en solution. De plus, cette composition s'est avérée phytotoxique à l'égard de certains végétaux aux concentrations efficaces. Enfin, son efficacité à l'égard de certaines maladies comme la Cercosporiose noire n'a pas été prouvée. On connaît également par le document WO 2005/074687 un procédé de traitement de maladies du bananier par application d'un antibiotique polyène, préférentiellement la natamycine. Cet antibiotique est mis en solution aqueuse puis pulvérisé sur les végétaux, ou ceux-ci sont baignés dans celle-ci. L'utilisation répétée d'un tel antibiotique présente un risque d'apparition de résistances et est donc un danger pour les écosystèmes.

Le document FR 2 953 369 décrit un concentré émulsionnable pour composition phytosanitaire, comprenant plus de 90% en poids d'au moins un mélange d'hydrocarbures de coupe de distillation supérieure à 250 ° C, comprenant moins de 20% en poids de n-paraffines et moins de 1% d'aromatiques, et de 1 à 4% en poids d'un mélange d'au moins deux tensioactifs TA1 et TA2, tels que TA2 est plus hydrophobe que TA1 et dont la HLB du mélange varie de 6 à 10. La teneur en poids du concentré en n-paraffines rapporté à la quantité totales de coupe hydrocarbure est donc strictement inférieure à 20%. En pratique, les isoparaffines y représentent plus de 90% des paraffines totales. L'activité phytosanitaire illustrée est une activité insecticide sur des cultures d'agrumes.

Les documents FR 2 984 690 et WO2013/092977 concernent l'utilisation d'une émulsion d'huile comme acaricide, insecticide et pour le traitement antifongique des tiges et des feuilles d'une plante lors de la croissance de celle-ci dans des espaces de culture confinés, à forte intensité de culture. L'émulsion comprend de l'eau et une huile de paraffine dérivée du pétrole et ayant un point d'ébullition de 200 ° C à 450 ° C, une viscosité inférieure ou égale à 20 mm²/s à 40 ° C, et une teneur en résidus insulfonables d'au moins 95% selon la norme ASTM D483. Les compositions sont testées pour leur activité acaricide et contre l'oïdium sur des cultures de rosiers. Les compositions décrites dans ce document comprennent un taux de n-paraffines très faible par rapport au taux d'iso-paraffines.

Dans le document WO2013/092977 l'huile paraffinique est introduite sous forme de concentré émulsionnable comprenant de l'eau.

FR 2 999 190 divulgue un procédé d'obtention d'une coupe hydrocarbonée comprenant les étapes de déparaffinage et d'hydrodéaromatisation. L'objectif de cet art antérieur est l'obtention de coupes à faible teneur en soufre et en aromatiques.

EP 0 255 871 décrit une coupe hydrocarbonée et son utilisation en tant que solvant pour encre. Ladite coupe hydrocarbonée doit être non-toxique, avec une odeur améliorée et posséder un haut pouvoir solvant des résines.

Aucun de ces documents ne décrit l'utilisation d'une coupe hydrocarbonée avec un taux de n-paraffines qui soit contrôlé par rapport au taux d'iso-paraffines, pour le traitement phytosanitaire de végétaux.

Il subsiste donc le besoin de disposer d'une composition de traitement des maladies cryptogamiques des plantes, qui soit efficace et non-toxique pour l'utilisateur, le végétal ou l'environnement. La profession est en recherche permanente de solutions pouvant améliorer la performance des compositions existantes.

La demanderesse a trouvé de manière surprenante que ce besoin peut être satisfait par une nouvelle composition d'huile phytosanitaire de traitement des cultures.

La présente invention a pour objectif de fournir une optimisation de la performance des traitements actuels des maladies cryptogamiques des plantes cultivées.

La présente invention a pour objectif de fournir une composition à l'efficacité accrue pour le traitement des maladies cryptogamiques des plantes. Notamment l'invention a pour objectif de fournir une composition utilisable comme adjuvant d'un principe actif dans le traitement des maladies cryptogamiques des plantes.

Notamment, la composition de l'invention est particulièrement adaptée pour améliorer les performances des compositions de traitement des plantes des régions tropicales et équatoriales.

En particulier, la composition de l'invention est particulièrement adaptée pour améliorer les performances des compositions de traitement des cultures en vue de réduire, limiter, prévenir, empêcher le développement de la cercosporiose noire ou maladie des raies noires du bananier, causée par le champignon ascomycète *Mycosphaerella fijiensis* et/ou de la cercosporiose jaune causée par le champignon ascomycète *Mycosphaerella musicola.*

Les compositions de l'invention ont pour avantage de ne pas bloquer la photosynthèse et de ne pas ralentir la croissance des plantes

La présente invention a également pour objectif de fournir une composition de traitement des maladies cryptogamiques ayant une viscosité adaptée, c'est-à-dire sans risque d'altération du métabolisme de la plante.

### RÉSUMÉ DE L'INVENTION

Ces objectifs sont atteints grâce à une nouvelle composition phytosanitaire.

L'invention concerne en premier lieu une composition phytosanitaire comprenant au moins une coupe hydrocarbonée paraffinique qui comprend des isoparaffines des paraffines normales et des naphtènes, la teneur en poids en naphtènes allant de 10 à 50 % par rapport au poids total de la composition, le ratio en poids des paraffines normales par rapport au poids des isoparaffines étant supérieur ou égal à 0,30, la coupe hydrocarbonée ayant un point d'écoulement supérieur ou égal à -5 °C selon la norme ASTM D97, dans un support adapté pour le traitement phytosanitaire de végétaux consistant en une émulsion huile-dans-eau, une émulsion eau-dans-huile, une dispersion ou une suspension de particules huileuses dans une phase aqueuse.

L'invention concerne également l'utilisation d'une coupe hydrocarbonée paraffinique qui comprend des isoparaffines, des paraffines normales et des naphtènes, la teneur en poids en naphtènes allant de 10 à 50 % par rapport au poids total de la composition, le ratio en poids des paraffines normales par rapport au poids des isoparaffines étant supérieur ou égal à 0,30, la coupe hydrocarbonée ayant un point d'écoulement supérieur ou égal à -5 °C selon la norme ASTM D97, pour le traitement phytosanitaire de végétaux.

L'invention concerne encore un procédé de traitement phytosanitaire de cultures comprenant au moins une étape d'application, de préférence par aspersion, pulvérisation ou épandage de la composition phytosanitaire selon l'invention.

L'invention concerne aussi une composition concentrée émulsionnable, utilisable pour la préparation d'une composition phytosanitaire selon l'invention, comprenant :
- au moins une coupe hydrocarbonée paraffinique qui comprend des isoparaffines des paraffines normales et des naphtènes, la teneur en poids en naphtènes allant de 10 à 50 % par rapport au poids total de la composition, le ratio en poids des paraffines normales par rapport au poids des isoparaffines étant supérieur ou égal à 0,30, la coupe hydrocarbonée ayant un point d'écoulement supérieur ou égal à -5 °C selon la norme ASTM D97, et
- au moins un agent émulsionnant.

Selon un mode de réalisation avantageux, la coupe hydrocarbonée comprend une teneur en poids en paraffines normales supérieure ou égale à 15 % et préférentiellement supérieure ou égale à 20 % par rapport au poids total de la coupe hydrocarbonée.

Selon un mode de réalisation avantageux, la coupe hydrocarbonée comprend une teneur en poids d'isoparaffines inférieure à 75 %, de préférence inférieure ou égale à 65 % et préférentiellement inférieure ou égale à 50 % par rapport au poids total de la coupe hydrocarbonée.

Selon un mode de réalisation avantageux, la coupe hydrocarbonée comprend une teneur en poids de composés naphténiques allant de 15 à 45 %, encore plus préférentiellement de 20 à 40 % par rapport au poids total de la coupe hydrocarbonée.

Selon un mode de réalisation avantageux, les composants de la coupe hydrocarbonée sont choisis parmi les chaînes hydrocarbonées comprenant de 15 à 30 atomes de carbone, de préférence de 16 à 27 atomes de carbone et préférentiellement de 17 à 25 atomes de carbone.

Selon un mode de réalisation avantageux, la coupe hydrocarbonée comprend une teneur en poids de résidus insulfonables supérieure ou égale à 92 %, de préférence supérieure ou égale à 95 %, préférentiellement supérieure ou égale à 99 % par rapport au poids total de la coupe hydrocarbonée.

Selon un mode de réalisation avantageux, la coupe hydrocarbonée a une température d'ébullition allant de 250 à 400 °C, de préférence de 260 à 390 °C et préférentiellement de 270 à 380 °C selon la norme ASTM D86.

Selon un mode de réalisation avantageux, la coupe hydrocarbonée a une viscosité cinématique à 40°C allant de 4 à 25 mm2/s, préférentiellement de 5 à 20 mm2/s et plus préférentiellement de 5 à 10 mm2/s selon la norme ASTM D445.

Selon un mode de réalisation avantageux, la coupe hydrocarbonée a un point d'écoulement supérieur ou égal à 0 °C et préférentiellement supérieur ou égal à 5 °C selon la norme ASTM D97.

Selon un mode de réalisation avantageux, la coupe hydrocarbonée représente une quantité allant de 1 à 99 % en poids par rapport au poids total de la composition.

Selon un mode de réalisation avantageux, la composition phytosanitaire est sous forme d'une émulsion huile-dans-eau ou d'une émulsion eau-dans-huile.

Selon un mode de réalisation avantageux, l'utilisation est pour une application par aspersion sur les cultures, notamment par pulvérisation ou épandage.

Selon un mode de réalisation avantageux, la coupe hydrocarbonée est formulée sous forme d'une émulsion huile-dans-eau ou d'une émulsion eau-dans-huile.

Selon un mode de réalisation avantageux, l'utilisation est destinée au traitement préventif et/ou curatif des maladies cryptogamiques des plantes.

Selon un mode de réalisation avantageux, l'utilisation est destinée au traitement préventif et/ou curatif des maladies cryptogamiques des plantes des régions tropicales et équatoriales.

Selon un mode de réalisation avantageux, l'utilisation est destinée au traitement préventif et/ou curatif des maladies cryptogamiques du bananier.

Selon un mode de réalisation avantageux, l'utilisation est destinée réduire, limiter, prévenir, empêcher le développement de la cercosporiose noire ou maladie des raies noires du bananier, causée par le champignon ascomycète *Mycosphaerella fijiensis* et/ou de la cercosporiose jaune causée par le champignon ascomycète *Mycosphaerella musicola.*

Selon un mode de réalisation avantageux, le procédé de l'invention comprend en outre l'application conjointe d'au moins un principe actif fongicide

### DESCRIPTION DETAILLÉE DE L'INVENTION

L'invention concerne l'utilisation d'une composition d'huile phytosanitaire comprenant au moins une coupe hydrocarbonée issue du pétrole brut ou une coupe hydrocarbonée issue de la conversion de la biomasse, pour le traitement des maladies cryptogamiques.

Elle concerne une composition phytosanitaire comprenant cette composition huileuse dans un support acceptable pour une application phytosanitaire.

A titre préliminaire on notera que, dans la description et les revendications suivantes, l'expression « compris entre » doit s'entendre comme incluant les bornes citées.

### Composition d'huile phytosanitaire :

La composition d'huile phytosanitaire selon l'invention comprend au moins une coupe hydrocarbonée d'origine pétrolière ou issue de la biomasse.

La composition d'huile phytosanitaire selon l'invention comprend de préférence une teneur en coupe hydrocarbonée allant de 50 à 100 %, de préférence de 70 à 100 %, plus préférentiellement de 80 à 100% et idéalement de 90 à 100 % en poids par rapport au poids total de la composition d'huile phytosanitaire.

Les autres composants de l'huile de protection phytosanitaire peuvent être : des principes actifs phytosanitaires oléosolubles, des huiles d'origine végétale ou animale, des tensioactifs.

Les huiles d'origine biologique appropriées sont par exemple l'huile de colza, l'huile de canola, l'huile de tall, l'huile de tournesol, l'huile de soja, l'huile de chanvre, l'huile d'olive, l'huile de lin, l'huile de moutarde, l'huile de palme, l'huile d'arachide, l'huile de ricin, l'huile de noix de coco, les graisses animales telles que le suif, les graisses alimentaires recyclées.
Notamment la composition d'huile phytosanitaire peut être sous forme d'un concentré émulsionnable : les tensioactifs sont introduits dans la composition huileuse de façon à permettre la production d'une émulsion par simple mélange de cette composition huileuse avec une phase aqueuse. Le concentré émulsionnable peut comprendre de petites quantités d'eau, des principes actifs phytosanitaires oléosolubles, des huiles d'origine végétale ou animale.

### Coupe hydrocarbonée :

La coupe hydrocarbonée mise en œuvre dans la composition selon l'invention peut encore être appelée solvant hydrocarboné. Avantageusement elle est essentiellement constituée de composants paraffiniques et de naphtènes.

La coupe hydrocarbonée mise en œuvre dans la composition selon l'invention a de préférence une teneur en poids en composés paraffiniques allant de 50 à 100 %, avantageusement de 50 à 90%, de préférence de 55 à 85 % et plus préférentiellement de 60 à 80 %. Ces paraffines sont généralement des mélanges de paraffines normales et d'isoparaffines.

La coupe hydrocarbonée mise en œuvre dans la composition selon l'invention comprend une teneur significative en paraffines normales. Par teneur significative, on entend que la teneur en poids de paraffines normales de la coupe hydrocarbonée est supérieure à 10 %, préférentiellement la teneur en paraffines normales de la coupe hydrocarbonée est supérieure ou égale à 15 % en poids et plus préférentiellement la teneur en paraffines normales de la coupe hydrocarbonée est supérieure ou égale à 20 % en poids, par rapport au poids total de la coupe hydrocarbonée.

La coupe hydrocarbonée mise en œuvre dans la composition selon l'invention comprend également une teneur en poids d'iso-paraffines inférieure à 80 %, avantageusement inférieure ou égale à 75%, de préférence inférieure ou égale à 65 %, préférentiellement inférieure ou égale à 50 %, par rapport au poids total de la coupe hydrocarbonée.

La coupe hydrocarbonée mise en œuvre dans la composition selon l'invention a également une teneur en poids de composés naphténiques allant de 0 à 50 %, avantageusement de 10 à 50%, de préférence de 15 à 45 %, encore plus préférentiellement de 20 à 40 %.

Selon un mode de réalisation préféré, la coupe hydrocarbonée comprend une teneur en poids, d'iso-paraffines inférieure ou égale à 70 %, de paraffines normales supérieure ou égale à 15 % et de naphtènes allant de 10 à 50%. Préférentiellement, la coupe hydrocarbonée comprend une teneur en poids d'iso-paraffines inférieure ou égale à 65 %, de paraffines normales supérieure ou égale à 15 % et de naphtènes allant de 15 à 45 %. Plus préférentiellement, la coupe hydrocarbonée comprend une teneur en poids d'iso-paraffines inférieure ou égale à 60 %, de paraffines normales supérieure ou égale à 20 % et de naphtènes allant de 20 à 40 %.

Avantageusement, les iso-paraffines, les paraffines normales et les naphtènes représentent au moins 90% en poids par rapport au poids total de la coupe hydrocarbonée, de préférence au moins 95% en poids, encore mieux au moins 98% et encore plus préférentiellement au moins 99 %.

Le ratio du poids des paraffines normales par rapport au poids des isoparaffines dans la coupe hydrocarbonée est supérieur ou égal à 0,30, de préférence supérieur ou égal à 0,35, encore mieux, supérieur ou égal à 0,4. La demanderesse a découvert qu'un ratio en poids paraffines normales/isoparaffines, supérieur aux ratios divulgués dans l'art antérieur fournit d'excellent résultats dans le traitement des maladies cryptogamiques.

La coupe hydrocarbonée mise en œuvre dans la composition selon l'invention est avantageusement à très basse teneur en aromatiques. Par très basse teneur en aromatiques, on entend, de préférence, une coupe hydrocarbonée comprenant une teneur en composés aromatiques inférieure ou égale à 1000 ppm, préférentiellement inférieure ou égale à 500 ppm, encore plus préférentiellement inférieure ou égale 300 ppm, mesurée par spectrométrie UV.

La coupe hydrocarbonée a également de préférence une teneur en soufre inférieure ou égale à 10 ppm, préférentiellement inférieure ou égale à 5 ppm et plus préférentiellement inférieure ou égale à 2 ppm.

La coupe hydrocarbonée mise en œuvre dans la composition selon l'invention a de préférence une viscosité cinématique à 40°C allant de 4 à 25 mm2/s, préférentiellement de 4 à 20 mm2/s et plus préférentiellement de 5 à 10 mm2/s selon la norme ASTM D445.

La coupe hydrocarbonée selon l'invention a un point d'écoulement selon la norme ASTM D97 supérieure ou égale à -5 °C, préférentiellement supérieure ou égale à 0 °C et plus préférentiellement supérieure ou égale à 5 °C.

La coupe hydrocarbonée selon l'invention comprend aussi une teneur en poids de résidus insulfonables selon la norme ASTM D483 supérieure ou égale à 92 %, de préférence supérieure ou égale à 95 % et plus préférentiellement supérieure ou égale à 99 %.

De telles compositions de coupes hydrocarbonées peuvent être obtenues de la façon suivante. La coupe hydrocarbonée selon l'invention est une coupe hydrocarbonée qui peut être issue de façon connue du pétrole brut ou de la biomasse.

De préférence, on entend par coupe hydrocarbonée au sens de l'invention, une coupe issue de la distillation du pétrole brut, de préférence issue de la distillation atmosphérique et/ou de la distillation sous vide du pétrole brut, de préférence issue de la distillation atmosphérique suivie de la distillation sous vide.

La coupe hydrocarbonée mise en œuvre dans la composition de l'invention est avantageusement obtenue par un procédé comprenant des étapes d'hydrotraitement, hydrocraquage ou craquage catalytique.

La coupe hydrocarbonée mise en œuvre dans la composition de l'invention est de préférence obtenue par un procédé comprenant des étapes de déaromatisation et éventuellement désulfuration.

La coupe hydrocarbonée selon l'invention n'est pas soumise à une étape de déparaffinage. Le déparaffinage est un procédé de traitement connu des coupes hydrocarbonées sans conversion consistant à enlever les paraffines et les cires microcristallines d'une charge ou à les convertir en composés de masse moléculaire plus faible et/ou de structure moléculaire différente. Les procédés de déparaffinage classiquement connus sont des procédés d'extraction au solvant ou d'hydrodéparaffinage. Lors de ces procédés les paraffines normales sont extraites ou transformées en iso-paraffines afin généralement d'obtenir un point d'écoulement plus bas. Par déparaffinage, on entend un procédé de traitement permettant d'obtenir une coupe hydrocarbonée comprenant une teneur en poids de paraffines normales inférieure à 10 %. Les procédés conduisant à un déparaffinage partiel de la coupe hydrocarbonée ne sont pas exclus de l'invention.

De préférence, la coupe hydrocarbonée obtenue après la ou les étapes de distillation est choisie parmi les coupes gazole ou huiles minérales. La coupe gazole est de préférence obtenue par un procédé comprenant des étapes d'hydrotraitement, d'hydrocraquage ou de craquage catalytique, suivi éventuellement des étapes de déaromatisation et éventuellement de désulfuration. La coupe minérale est de préférence obtenue par un procédé comprenant des étapes de distillation sous vide, extraction solvant et éventuellement déparaffinage partiel et hydrotraitement ou hydrocraquage.

La coupe hydrocarbonée peut être un mélange de coupes hydrocarbonées ayant subi les étapes décrites ci-dessus.

La coupe hydrocarbonée mise en œuvre dans la composition de l'invention peut également être issue de la conversion de la biomasse.

Par issue de la conversion de la biomasse, on entend une coupe hydrocarbonée produite à partir de matières premières d'origine biologique choisies de préférence parmi les huiles végétales, les graisses animales, les huiles de poisson et leurs mélanges. Les matières premières d'origine biologique appropriées sont par exemple l'huile de colza, l'huile de canola, l'huile de tall, l'huile de tournesol, l'huile de soja, l'huile de chanvre, l'huile d'olive, l'huile de lin, l'huile de moutarde, l'huile de palme, l'huile d'arachide, l'huile de ricin, l'huile de noix de coco, les graisses animales telles que le suif, les graisses alimentaires recyclées, les matières premières issues du génie génétique, et les matières premières biologiques produites à partir de microorganismes tels que les algues et les bactéries.

De préférence, La coupe hydrocarbonée d'origine biologique est obtenue par un procédé comprenant des étapes d'hydro désoxygénation (HDO) et d'isomérisation. L'étape d'hydro désoxygénation (HDO) conduit à la décomposition des structures des esters biologiques ou des constituants triglycérides, à l'élimination des composés oxygénés, phosphorés et soufrés et à l'hydrogénation des liaisons oléfiniques. Le produit issu de la réaction d'hydro désoxygénation est ensuite isomérisé. Une étape de fractionnement peut de préférence suivre les étapes d'hydro désoxygénation et d'isomérisation.

Les fractions d'intérêt sont ensuite soumises à des étapes d'hydrotraitement puis de distillation afin obtenir les spécifications de la coupe hydrocarbonée souhaitée selon l'invention.

La coupe hydrocarbonée peut être un mélange de coupe hydrocarbonée issue de la distillation du pétrole brut et / ou de la conversion de la biomasse.

De préférence la coupe hydrocarbonée est une coupe hydrocarbonée issue de la distillation du pétrole brut.

Avantageusement la coupe hydrocarbonée est une coupe hydrocarbonée hydrogénée.

La coupe hydrocarbonée mise en œuvre dans la composition de l'invention est avantageusement une coupe hydrocarbonée ayant un intervalle de distillation ID (en °C) allant de 250 à 400 °C, de préférence de 260 à 390 °C et encore plus préférentiellement de 270 à 380 °C mesuré selon la norme ASTM D86. De préférence, la différence entre le point d'ébullition initial et le point d'ébullition final est inférieure ou égale à 100 °C. La coupe hydrocarbonée peut comprendre une ou plusieurs fractions d'intervalles de distillation compris dans les intervalles décrits ci-dessus.

Avantageusement, la coupe hydrocarbonée mise en œuvre dans la composition phytosanitaire selon l'invention est totalement saturée. De préférence, les composants de la coupe hydrocarbonée sont choisis parmi les chaînes hydrocarbonées comprenant comprenant de 15 à 30 atomes de carbone, préférentiellement de 16 à 27 atomes de carbone, plus préférentiellement de 17 à 25 atomes de carbone.

Selon un premier mode de réalisation, la coupe hydrocarbonée présente de préférence :
- une température d'ébullition allant de 250 à 400°C, de préférence de 260 à 390°C et encore plus préférentiellement de 270 à 380°C, mesurée selon la norme ASTM D86,
- une viscosité cinématique allant de 4 à 25 mm2/s, préférentiellement de 5 à 20 mm2/s et plus préférentiellement de 5 à 10 mm2/s selon la norme ASTM D445, et

Selon un second mode de réalisation, la coupe hydrocarbonée présente de préférence :
- une viscosité cinématique allant de 4 à 25 mm²/s, préférentiellement de 5 à 20 mm²/s et plus préférentiellement de 5 à 10 mm²/s selon la norme ASTM D445, et
- un point d'écoulement selon la norme ASTM D97 supérieure ou égale à -5 °C, préférentiellement supérieure ou égale à 0 °C et plus préférentiellement supérieure ou égale à 5 °C.

Selon un mode de réalisation préférentiel, la coupe hydrocarbonée présente :
- une température d'ébullition allant de 250 à 400°C, de préférence de 260 à 390°C et encore plus préférentiellement de 270 à 380°C, mesurée selon la norme ASTM D86,
- une viscosité cinématique allant de 4 à 25 mm2/s, préférentiellement de 5 à 20 mm2/s et plus préférentiellement de 5 à 10 mm2/s selon la norme ASTM D445, et
- un point d'écoulement selon la norme ASTM D97 supérieure ou égale à -5 °C, préférentiellement supérieure ou égale à 0 °C et plus préférentiellement supérieure ou égale à 5 °C.

### Composition phytosanitaire

L'invention concerne une composition comprenant la composition huileuse dans un support adapté pour une application phytosanitaire en vue du traitement de végétaux.

Par « support adapté pour le traitement phytosanitaire de végétaux » on entend un support qui serve de véhicule pour le traitement des végétaux, notamment par aspersion, épandage ou tout autre moyen permettant de traiter les végétaux. Ce support doit être non toxique pour la plante et pour son environnement, notamment pour l'homme.

Le support adapté pour une application phytosanitaire consiste en une émulsion huile-dans-eau, une émulsion eau-dans-huile, une dispersion ou une suspension de particules huileuses dans une phase aqueuse.

Outre une phase aqueuse, la composition phytosanitaire peut comprendre des tensioactifs permettant de former une émulsion de la phase huileuse dans la phase aqueuse, comme par exemple des tensioactifs non-ioniques, tels que les acides gras polyalcoxylé, les esters d'acides gras et de sorbitan, les esters d'acides gras et de sorbitan (poly)alcoxylés, les alkylphénol alcoxylés, les alcools gras alcoxylés, les esters d'acides gras et de glycérol.... Elle peut comprendre des polymères permettant la stabilisation de l'émulsion ou la dispersion ou de la suspension.

La phase aqueuse peut comprendre des sels, des principes actifs phytosanitaires hydrosolubles.

### Additifs :

La composition d'huile phytosanitaire selon l'invention peut également être mise en mélange avec tout autres produits phytosanitaires (insecticides et/ou fongicides) ainsi qu'avec des adjuvants (émulsionnants, mouillants, stabilisants, etc...) habituellement utilisés dans le domaine phytosanitaire. Bien entendu, l'homme du métier veillera à choisir le ou les éventuels autres adjuvants ou additifs de la composition selon l'invention de manière telle que les propriétés avantageuses attachées intrinsèquement à la composition phytosanitaire conforme à l'invention ne soient pas ou substantiellement pas, altérées par l'addition envisagée.

Sans se limiter à ceux-ci et à titre d'exemple, on peut citer comme fongicides utilisables avec la composition phytosanitaire selon l'invention, les produits de contact appartenant à la famille des dithiocarbamates comme le Mancozèbe, les produits systémiques de la famille des strobilurines (Azoxystrobine, Pyraclostrobine, Trifloxystrobine), ceux de la famille des carboximides (fluoryram, izopyrazam), des amines (Fenpropimorph, Tridemorph, Spiroxamine), des Anilino-Pyrimidines (Pyrimethanil), les inhibiteurs de succinate déshydrogénase (Boscalid) et les inhibiteurs de la déméthylation des stérols (Difénoconazole, Epoxyconazole, Fenbuconazole, Propiconazole, Tebuconazole).

Suivant le caractère hydrophile ou lipophile de ces additifs, ceux-ci seront introduits dans la phase aqueuse ou dans la phase huileuse.

Avantageusement, la composition de traitement comprend une teneur en composition d'huile phytosanitaire selon l'invention allant de 1 à 99 % en masse rapportée à la masse totale de la composition, avantageusement de 10 à 80%, et plus préférentiellement de 15 à 70 %. Le reste de la composition est constitué, de façon non limitative, de la phase aqueuse, d'éventuels additifs, de tensioactifs, de polymères, de principes actifs phytosanitaires, notamment des fongicides. Ces derniers, lorsqu'ils sont utilisés dans les compositions de l'invention, le sont aux doses habituellement recommandées en fonction du type de plante et de la gravité de la maladie.

### Concentré émulsionnable

De préférence le concentré émulsionnable comprend une teneur en composition d'huile phytosanitaire selon l'invention allant de 50 à 99 % en masse, avantageusement de 70 à 95% en masse, rapportée à la masse totale du concentré.

Parmi les autres composants du concentré émulsionnable on peut citer de façon non exhaustive : des tensio-actifs émulsionnants, des agents de stabilisation, des sels, des principes actifs phytosanitaires et éventuellement de l'eau.

Les tensio-actifs émulsionnants sont choisis pour permettre la formation d'une émulsion eau-dans-huile ou d'une émulsion huile-dans-eau, une dispersion ou une suspension de particules huileuses dans une phase aqueuse.

De préférence, le concentré émulsionnable comprend au moins un tensioactif permettant de former une émulsion de la phase huileuse dans la phase aqueuse, comme par exemple des tensioactifs non-ioniques, tels que les acides gras polyalcoxylé, les esters d'acides gras et de sorbitan, les esters d'acides gras et de sorbitan (poly)alcoxylés, les alkylphénol alcoxylés, les alcools gras alcoxylés, les esters d'acides gras et de glycérol....

Parmi les agents de stabilisation, on peut citer des polymères permettant la stabilisation de l'émulsion ou de la dispersion ou de la suspension.

Lorsqu'elle est présente, la phase aqueuse du concentré peut comprendre des sels, des principes actifs phytosanitaires hydrosolubles tels qu'ils ont été décrits ci-dessus.

### Utilisation de la composition :

L'invention a encore pour objet l'utilisation de la composition d'huile phytosanitaire telle que définie ci-dessus pour le traitement curatif et/ou préventif des maladies cryptogamiques des plantes. Elle concerne également l'utilisation de la composition phytosanitaire décrite ci-dessus, préférentiellement une émulsion eau-dans-huile ou huile-dans-eau, pour le traitement curatif et/ou préventif des maladies cryptogamiques des plantes.

L'invention concerne plus particulièrement le traitement préventif et/ou curatif des maladies cryptogamiques des plantes des régions tropicales et équatoriales.

L'invention concerne plus particulièrement le traitement préventif et/ou curatif des maladies cryptogamiques du bananier.

En particulier, la composition de l'invention est particulièrement adaptée au traitement des cultures en vue de réduire, limiter, prévenir, empêcher le développement de la cercosporiose noire ou maladie des raies noires du bananier, causée par le champignon ascomycète *Mycosphaerella fijiensis* et/ou de la cercosporiose jaune causée par le champignon ascomycète *Mycosphaerella musicola.*

Selon une première variante, la composition huileuse, ou la composition phytosanitaire, est utilisée seule dans le cadre de cette application.

Selon une autre variante, préférée, de l'invention, la composition huileuse, ou la composition phytosanitaire, est utilisée conjointement avec un traitement fongicide mettant en œuvre au moins un principe actif fongicide. Dans ce cas la composition huileuse, ou la composition phytosanitaire, a pour fonction de servir d'adjuvant au principe actif fongicide et de renforcer son action à l'encontre des maladies cryptogamiques.

En fonction du mode d'administration de la composition de l'invention, notamment conjointement avec, ou en l'absence d'un traitement conjoint par un principe actif fongicide, les quantités et les fréquences d'administration sont adaptées par l'homme du métier.

### Procédé de traitement phytosanitaire :

Enfin, l'invention couvre aussi un procédé de traitement phytosanitaire comprenant au moins une étape d'aspersion de la plante à traiter, de préférence par épandage ou par pulvérisation de la composition d'huile phytosanitaire selon l'invention sur les plantes à traiter. Le traitement est avantageusement appliqué sur les parties aériennes des plantes.

Le taux de traitement est avantageusement de 0,5 à 20 litres d'huile phytosanitaire par hectare, de préférence de 1 à 15 litres d'huile phytosanitaire par hectare. En fonction de la dilution de la composition, la quantité de composition phytosanitaire pulvérisée, aspergée ou épandue est adaptée.

Le traitement est avantageusement répété à des intervalles de 6 à 9 jours, tout au long du cycle végétatif.

Selon une première variante de ce procédé, la composition huileuse, ou la composition phytosanitaire, est administrée seule dans le cadre de ce traitement.

Selon une autre variante, préférée, de l'invention, la composition huileuse, ou la composition phytosanitaire, est administrée conjointement avec un traitement fongicide mettant en œuvre au moins un principe actif fongicide.

Par conjointement on entend que le principe actif peut être dilué dans la composition de l'invention, mais aussi qu'il peut être administré dans une autre composition, qui est appliquée simultanément ou de façon alternée avec la composition de l'invention.

### EXEMPLES

Dans la suite de la présente description, des exemples sont donnés à titre illustratif de la présente invention et ne visent en aucun cas à en limiter la portée.

Différentes compositions phytosanitaires, selon l'invention, et classiquement retrouvées dans le domaine de l'invention, ont été évaluées. Cette évaluation porte à la fois sur l'efficacité fongistatique et les propriétés physico chimiques de ces compositions phytosanitaires.

Dans la série de tests décrite ci-dessous, l'effet du taux de paraffines normales contenues dans l'huile a spécifiquement été évalué.

Différentes huiles hydrocarbonées ont ainsi été évaluées.
- L'huile hydrocarbonée A est une huile hydrocarbonée selon l'invention.
- L'huile hydrocarbonée B est une huile hydrocarbonée comparative. C'est une huile minérale paraffinique issue du raffinage du pétrole et obtenue par distillation atmosphérique, distillation sous vide, hydrocraquage et hydrotraitement.
- L'huile hydrocarbonée C est une huile minérale comparative utilisée pour la formulation de produits phytosanitaires couramment retrouvés sur le marché sous le nom commercial Banole HV. Cette huile hydrocarbonée est une huile minérale largement utilisée dans le traitement de la cercosporiose. Elle est issue du raffinage du pétrole et est obtenue par un procédé comprenant entre autre une étape de déparaffinage permettant d'obtenir des points d'écoulement bas.
- L'huile hydrocarbonée D est une huile minérale comparative utilisée pour la formulation de produits phytosanitaires couramment retrouvés sur le marché sous le nom commercial Spraytex M100. Cette huile hydrocarbonée est issue du raffinage du pétrole et est obtenue par un procédé comprenant entre autre une étape de déparaffinage permettant d'obtenir des points d'écoulement bas.

Les huiles comparatives C et D sont classiquement retrouvées dans le marché des compositions phytosanitaires.

Le tableau 1 regroupe les propriétés physico chimiques des différentes huiles hydrocarbonées formulées dans les compositions phytosanitaires évaluées.

Les % sont donnés en masse par rapport à la masse totale des composants

**Tableau1**

| | | Huiles hydrocarbonées | | | |
|---|---|---|---|---|---|
| Caractéristiques | Normes | A (selon l'invention) *DEV1781* | B (exemple comparatif) *G400H Banole Oudale* | C (exemple comparatif) *Calumet, Banole HV* | D (exemple comparatif) *Spraytex M100SL* |
| Soufre (%) | ASTM D2622 | < 0,001 | < 0,001 | < 0,001 | < 0,001 |
| paraffines total (%) | GC2D | 69 | 75 | 65 | 65 |
| iso paraffines (%) | GC2D | 46 | 59 | 61 | 64 |
| n-paraffines (%) | GC2D | 23 | 16 | 1 | 1 |
| naphténiques (%) | GC2D | 31 | 25 | 35 | 35 |
| Ratio normales/iso paraffines | - | 0,50 | 0,27 | 0,016 | 0,015 |
| Longueur de chaine hydrocarbonée | - | C18-C24 | C18-C22 | C20-C27 | C21-C30 |
| Point d'ébullition initial (°C) | ASTM D86 | 300 | 300 | 330 | 345 |
| Point d'ébullition final (°C) | | 370 | 350 | 395 | 415 |
| Masse volumique à 15°C (kg/m3) | EN ISO 12185 | 822 | 817 | 857 | 852 |
| Point éclair (°C) | ASTM D93 | 160 | 158 | 186 | 200 |
| Point d'aniline (°C) | ASTM D611 | 103 | 101 | 98 | 98 |
| Point d'écoulement (°C) | ASTM D97 | 9 | 0 | -27 | -15 |
| Viscosité Cinématique à 40°C (cSt) | ASTM D445 | 8 | 6 | 15 | 19 |
| Résidus insulfonables (%) | ASTM D483 | > 99 | > 99 | 95 | > 99 |

### Compositions phytosanitaires :

Différentes compositions phytosanitaires ont été formulées afin que soit évaluée leur efficacité fongistatique.

Les différentes compositions phytosanitaires exemplifiées comprennent les huiles hydrocarbonées A à D selon le tableau 1. La composition 1 est une composition phytosanitaire selon l'invention. Les compositions 2 et 3 sont des compositions phytosanitaires comparatives. Elles comprennent les mêmes proportions d'huile hydrocarbonée que la composition 1 selon l'invention. La composition 4 est une composition comparative de référence comprenant l'huile hydrocarbonée D. Cette composition est retrouvée sur le marché des produits phytosanitaires destinés au traitement de la cercosporiose du bananier et commercialisée sous le nom Spraytex M 100 par la société Chevron. La composition 5 est une composition phytosanitaire selon l'invention qui se distingue de la composition 1 en ce qu'elle comprend 20% en moins d'huile hydrocarbonée A par rapport à la composition 1, le complément étant de l'eau.

Le tableau 2 ci-dessous indique quelle huile est comprise dans chaque formulation de composition phytosanitaire exemplifiée.

**Tableau 2**

| | Compositions phytosanitaires | | | | |
|---|---|---|---|---|---|
| | 1 (selon l'invention) *DEV1781* | 2 (exemple comparatif) *G400H* | 3 (exemple comparatif) *Calumet*/ *Banole HV* | 4 (exemple comparatif) *Spray tex* | 5 (selon l'invention) *DEV1781* |
| Huile hydrocarbonée A | X | | | | x |
| Huile hydrocarbonée B | | X | | | |
| Huile hydrocarbonée C | | | X | | |
| Huile hydrocarbonée E | | | | X | |

### Evaluation des compositions phytosanitaires

### Méthode d'évaluation :

L'évaluation a été faite sur différentes parcelles de bananiers sujets à la cercosporiose.

Chaque parcelle est traitée par aspersion mécanique des compositions mises en émulsion. Ces émulsions de type huile-dans-l'eau sont formulées grâce à un émulsifiant classiquement utilisé dans le domaine de l'invention, nommé Imbirex ® CR80SL commercialisé par la société Bayer CropScience. Ce type d'émulsifiant n'a pas d'action directe active sur le traitement des plantations.

Les traitements sont répétés à une fréquence et selon une durée détaillées pour chaque phase de test et sont appliqués de manière aléatoire sur les parcelles. Chaque plant a fait l'objet d'une observation détaillée.

Sur les bananiers observés, les valeurs des quatre paramètres suivants ont été déterminées chaque semaine :
- le nombre total de feuille par arbre,
- la plus jeune feuille à tirets. Les tirets sont des lésions de forme allongée et de couleur rouge-maronnée.
- la plus jeune feuille nécrosée. Les nécroses sont formées par l'élargissement et la coalescence des tirets.
- l'indice de sévérité de la maladie.

Les feuilles des bananiers sont classées en fonction de leur date d'apparition sur le plant et des nœuds sur les troncs. La plus jeune feuille étant la feuille numéro 1. Les feuilles du haut du tronc étant les plus jeunes et les feuilles les plus basses les plus âgées.

La réponse au traitement est évaluée grâce à l'échelle de Stover modifiée selon Gauhl décrite dans le tableau 3.

**Tableau 3**

| **Stade de la maladie** | **Description** |
|---|---|
| 0 | Aucun symptôme |
| 1 | De plusieurs tirets à 10 nécroses maximum |
| 2 | De 11 zones nécrosées à 5 % de la surface de la feuille infectée |
| 3 | 6-15 % de la surface de la feuille infectée |
| 4 | 16-33% de la surface de la feuille infectée |
| 5 | 34-50 % de la surface de la feuille infectée |
| 6 | Plus de 50 % de la surface de la feuille infectée |

L'apparition des premiers symptômes est corrélée avec la sévérité de l'infection : plus la feuille infectée est jeune et plus le niveau de sévérité de l'infection est important

### Phase de test 1 : Relation teneur en paraffines normales /efficacité biologique

Cette série de tests est réalisée pour démontrer l'efficacité de la composition selon l'invention et l'effet de la teneur en paraffines normales de la composition.

Cette série de tests est réalisée sur de grandes parcelles de cultures afin de reproduire spécifiquement la pratique commerciale. Les compositions évaluées sont associées à différents types de fongicides. Les fongicides sont utilisés en alternance dans les compositions afin d'éviter tout phénomène de résistance ou d'accoutumance.

Les essais sont réalisés sur 4 parcelles de 780m², à raison d'une parcelle par composition phytosanitaire, chacune comprenant 105 plants. Chaque parcelle est entourée de bordures plantées de bananiers de type *Musa textilis* résistant à la cercosporiose noire, de façon à éviter les contaminations entre parcelles.

Dans cette série de test, les 4 parcelles sont traitées selon un programme correspondant à la pratique commerciale. Ainsi sont comparées deux huiles non déparaffinées dans les compositions 1 et 5 selon l'invention et la composition 2 ainsi que deux huiles déparaffinées dans les compositions 3 et 4. L'huile non déparaffinée des compositions 1 et 5 selon l'invention contient un taux élevé de paraffines normales comparativement à la composition 2.

### Formulation et traitements des parcelles :

Pour chaque hectare de plantation 23 litres de composition phytosanitaire formulées comme ci-dessous sont répandus par aspersion :
- 3, 5 ou 7 litres de composition d'huile phytosanitaire A, B, C ou D
- cette quantité est réduite à 2,4, 4 ou 5,6 litres de composition d'huile phytosanitaire A pour la composition 5
- 16, 18 ou 20 litres d'eau (complément pour atteindre 23 litres au total)
- la composition 5 comprend 17,4, 19 ou 20,6 litres d'eau (complément pour atteindre 23 litres au total)
- 0,05 litres d'émulsifiant

La quantité de composition d'huile phytosanitaire A, B, C ou D est adaptée par l'homme du métier à chaque application, notamment en fonction des conditions climatiques.

Le taux de traitement est donc de 3, 5 ou 7 litres de composition d'huile phytosanitaire par hectare en mélange dans de l'eau avec 1% d'émulsifiant pour un volume global de traitement de 23 litres de composition phytosanitaire par hectare pour les compositions 1 à 4.

Le taux de traitement est de 2,4, 4 ou 5,6 litres de composition d'huile phytosanitaire par hectare en mélange dans de l'eau avec 1% d'émulsifiant pour un volume global de traitement de 23 litres de composition phytosanitaire par hectare pour la composition 5. Les différents fongicides ont été intégrés dans les compositions phytosanitaires en alternance pour éviter les développements de résistance. La liste de ces fongicides, ainsi que leur fréquence d'utilisation sont les suivantes :
- triazoles : 9 jours
- amines et aniloprimidines : 7 jours
- composés protecteurs (mancozeb) : 6 jours

**Tableau 4 : nature et quantité de composition fongicide commerciale appliquée**

| **Nom commercial** | **Principe actif** | **Famille chimique** | **Quantité de produit commercial appliquée (l/ha)** |
|---|---|---|---|
| Opus 12.5 SC | Epoxiconazole | DMI (inhibiteurs de déméthylation) | 0.8 |
| Sico 250 EC | Difenoconazole | DMI | 0.4 |
| Silvacur 30 EC | Tebuconazole + triadimenol | DMI + Amines | 0.4 |
| Tilt 250 EC | Propiconazole | DMI | 0.4 |
| Volley 88 OL | Fenpropimorph | Amines | 0.5 |
| Calixin 86 OL | Tridemorph | Amines | 0.5 |
| Impulse 80 EC | Spiroxamine | Amines | 0.4 |
| Siganex 60 SC | Pyrimethanil | AP (Anilino-Pyrimidines) | 0.5 |
| Cumora 50 SC | Boscalid | (SDHI) inhibiteurs de succinate deshydrogénase | 0.3 |
| Dithane 60 SC | Mancozeb | Dithiocarbamates | 2.0 |

Les traitements sont répétés par intervalle de traitement fongistatique selon l'ordre indiqué ci-dessus. Les quantités de fongicide administrées sont exposées dans le tableau 4. La fréquence de traitement est d'une application tous les 6 à 9 jours. Chaque bananier a fait l'objet d'une observation détaillée pour chacune des émulsions pendant une durée totale de 40 semaines. A l'issue de ces 40 semaines l'état des plants a été analysé puis les plants ont été coupés. Une seconde campagne de traitement a repris deux semaines après la coupe de la première génération de plants, pour une durée de 38 semaines. Une évaluation de l'état des plants a été réalisée après 15 semaines de la seconde campagne de traitement.

Remarque : la séquence d'application des fongicides est adaptée par l'homme du métier, en se fondant sur les recommandations du comité d'action contre la résistance aux fongicides (FRAC pour Fungicide Résistance Action Commitee) : http://www.frac.info/working-group/banana-group/banana-frac-guidelines-2014-summary-table

### Résultats :

Le tableau 5 indique la moyenne des résultats obtenus dans la première campagne de traitement (plants de première génération) pour chaque paramètre évalué.

**Tableau 5**

| **Traitements** | **Age de la plus jeune feuille à tirets** | **Age de la plus jeune feuille nécrosée** | **Nombre de feuilles au total** | **Sévérité** |
|---|---|---|---|---|
| Composition1 *DEV1781* | 7.4 | 9.8 | 13.5 | 0.16 |
| Composition 2 *G400H* | 6.6 | 8.4 | 13.5 | 0.42 |
| Composition 3 *Calumet*/*banole HV* | 6.9 | 8.6 | 13.5 | 0.34 |
| Composition 4 *Spray tex* | 6.5 | 8.2 | 13.4 | 0.49 |

Les indices de sévérité montrent que les résultats obtenus sur les bananiers traités avec la composition 1 selon l'invention, qui comprend un taux plus élevé de paraffines normales, sont largement meilleurs que ceux des autres compositions. En effet, la sévérité de la maladie pour les bananiers traités avec la composition 1 selon l'invention est plus de 2 fois moins importante qu'avec la composition 3 et de 2,5 à 3 fois moins importante qu'avec les compositions 2 et 4. Le traitement des parcelles avec la composition 1 selon l'invention permet donc de réduire les effets du champignon et de garantir la production des cultures.

Le tableau 6 indique la moyenne des résultats obtenus dans la seconde campagne de traitement (plants de seconde génération) pour chaque paramètre évalué.

**Tableau 6**

| **Traitements** | **Age de la plus jeune feuille à tirets** | **Age de la plus jeune feuille nécrosée** | **Nombre de feuilles au total** | **Sévérité** |
|---|---|---|---|---|
| Composition1 | 7.8 | 9.3 | 12.1 | 0.34 |
| Composition 5 *DEV1781* | 7.0 | 8.6 | 11.8 | 0.50 |
| Composition 3 *Calumet*/*banole HV* | 7.1 | 8.7 | 12.4 | 0.53 |
| Composition 4 *Spray tex* | 6.4 | 8.0 | 11.8 | 0.69 |

Les indices de sévérité montrent que les résultats obtenus sur les bananiers traités avec la composition 1 selon l'invention, qui comprend un taux plus élevé de paraffines normales, sont largement meilleurs que ceux des autres compositions. La composition 5 qui comprend une teneur moindre en huile hydrocarbonée selon l'invention présente des résultats tout à fait acceptables en termes de contrôle des effets du champignon.

## Revendications

1. Composition phytosanitaire comprenant au moins une coupe hydrocarbonée paraffinique qui comprend des isoparaffines, des paraffines normales et des naphtènes, la teneur en poids en naphtènes allant de 10 à 50 % par rapport au poids total de la composition, le ratio en poids des paraffines normales par rapport au poids des isoparaffines étant supérieur ou égal à 0,30, la coupe hydrocarbonée ayant un point d'écoulement supérieur ou égal à -5 °C selon la norme ASTM D97, dans un support adapté pour le traitement phytosanitaire de végétaux, consistant en une émulsion huile-dans-eau, une émulsion eau-dans-huile, une dispersion ou une suspension de particules huileuses dans une phase aqueuse.

2. Composition phytosanitaire selon la revendication 1 dans laquelle la coupe hydrocarbonée comprend une teneur en poids en paraffines normales supérieure ou égale à 15 % et préférentiellement supérieure ou égale à 20 % par rapport au poids total de la coupe hydrocarbonée.

3. Composition phytosanitaire selon l'une quelconque des revendications précédentes dans laquelle la coupe hydrocarbonée comprend une teneur en poids d'isoparaffines inférieure à 75 %, de préférence inférieure ou égale à 65 % et préférentiellement inférieure ou égale à 50 % par rapport au poids total de la coupe hydrocarbonée.

4. Composition phytosanitaire selon l'une quelconque des revendications précédentes dans laquelle la coupe hydrocarbonée comprend une teneur en poids de composés naphténiques allant de 15 à 45 %, encore plus préférentiellement de 20 à 40 % par rapport au poids total de la coupe hydrocarbonée.

5. Composition phytosanitaire selon l'une quelconque des revendications précédentes dans laquelle les composants de la coupe hydrocarbonée sont choisis parmi les chaînes hydrocarbonées comprenant de 15 à 30 atomes de carbone, de préférence de 16 à 27 atomes de carbone et préférentiellement de 17 à 25 atomes de carbone.

6. Composition phytosanitaire selon l'une quelconque des revendications précédentes dans laquelle la coupe hydrocarbonée comprend une teneur en poids de résidus insulfonables supérieure ou égale à 92 %, de préférence supérieure ou égale à 95 %, préférentiellement supérieure ou égale à 99 % par rapport au poids total de la coupe hydrocarbonée.

7. Composition phytosanitaire selon l'une quelconque des revendications précédentes dans laquelle la coupe hydrocarbonée a une température d'ébullition allant de 250 à 400 °C, de préférence de 260 à 390 °C et préférentiellement de 270 à 380 °C selon la norme ASTM D86.

8. Composition phytosanitaire selon l'une quelconque des revendications 1 à 7 dans laquelle la coupe hydrocarbonée a une viscosité cinématique à 40°C allant de 4 à 25 mm²/s, préférentiellement de 5 à 20 mm²/s et plus préférentiellement de 5 à 10 mm²/s selon la norme ASTM D445.

9. Composition phytosanitaire selon l'une quelconque des revendications 1 à 7 dans laquelle la coupe hydrocarbonée a un point d'écoulement supérieur ou égal à 0 °C et préférentiellement supérieur ou égal à 5 °C selon la norme ASTM D97.

10. Composition phytosanitaire selon l'une quelconque des revendications précédentes qui est sous forme d'une émulsion huile-dans-eau ou d'une émulsion eau-dans-huile.

11. Composition concentrée émulsionnable, utilisable pour la préparation d'une composition phytosanitaire selon l'une des revendications précédentes, comprenant :
• au moins une coupe hydrocarbonée paraffinique qui comprend des isoparaffines, des paraffines normales et des naphtènes, la teneur en poids en naphtènes allant de 10 à 50 % par rapport au poids total de la composition, le ratio en poids des paraffines normales par rapport au poids des isoparaffines étant supérieur ou égal à 0,30, la coupe hydrocarbonée ayant un point d'écoulement supérieur ou égal à -5 °C selon la norme ASTM D97 et
• au moins un agent émulsionnant.

12. Utilisation d'une coupe hydrocarbonée paraffinique qui comprend des isoparaffines, des paraffines normales et des naphtènes, la teneur en poids en naphtènes allant de 10 à 50 % par rapport au poids total de la composition, le ratio en poids des paraffines normales par rapport au poids des isoparaffines étant supérieur ou égal à 0,30, la coupe hydrocarbonée ayant un point d'écoulement supérieur ou égal à -5 °C selon la norme ASTM D97, pour le traitement phytosanitaire de végétaux.

13. Utilisation selon la revendication 12, pour une application par aspersion sur les cultures, notamment par pulvérisation ou épandage.

14. Utilisation selon l'une quelconque des revendications 12 et 13, pour le traitement préventif et/ou curatif des maladies cryptogamiques des plantes, de préférence des plantes des régions tropicales et équatoriales.

15. Utilisation selon la revendication 14 pour le traitement préventif et/ou curatif des maladies cryptogamiques du bananier.

16. Utilisation selon la revendication 15, en vue de réduire, limiter, prévenir, empêcher le développement de la cercosporiose noire ou maladie des raies noires du bananier, causée par le champignon ascomycète *Mycosphaerella fijiensis* et/ou de la cercosporiose jaune causée par le champignon ascomycète *Mycosphaerella musicola.*

17. Procédé de traitement phytosanitaire de cultures comprenant au moins une étape d'application, de préférence par aspersion, pulvérisation ou épandage de la composition phytosanitaire selon l'une quelconque des revendications 1 à 10.

18. Procédé selon la revendication 17 qui comprend en outre l'application conjointe d'au moins un principe actif fongicide.

## Patentansprüche

1. Pflanzenschutzzusammensetzung, die mindestens eine paraffinartige Kohlenwasserstofffraktion umfasst, welche Isoparaffine, Normalparaffine und Cycloalkane umfasst, wobei der gewichtsmäßige Gehalt an Cycloalkanen im Bereich von 10 bis 50 % liegt, unter Bezugnahme auf das Gesamtgewicht der Zusammensetzung, das Gewichtsverhältnis der Normalparaffine unter Bezugnahme auf das Gewicht der Isoparaffine mindestens 0,30 beträgt, die Kohlenwasserstofffraktion einen Pourpoint hat, der höher oder gleich -5 °C gemäß der Norm ASTM D97 ist, in einem Trägerstoff, welcher zur pflanzenschützenden Behandlung von Pflanzen geeignet ist und welcher in einer Öl-in-Wasser-Emulsion, einer Wasser-in-ÖI-Emulsion, einer Dispersion oder einer Suspension von ölartigen Partikeln in einer wässrigen Phase besteht.

2. Pflanzenschutzzusammensetzung nach Anspruch 1, wobei die Kohlenwasserstofffraktion einen gewichtsmäßigen Gehalt an Normalparaffinen von mindestens 15 %, und vorzugsweise von mindestens 20 % umfasst, unter Bezugnahme auf das Gesamtgewicht der Kohlenwasserstofffraktion.

3. Pflanzenschutzzusammensetzung nach einem beliebigen der vorhergehenden Ansprüche, wobei die Kohlenwasserstofffraktion einen gewichtsmäßigen Gehalt an Isoparaffinen von weniger als 75 %, vorzugsweise von höchstens 65 % und bevorzugt von höchstens 50 % umfasst, unter Bezugnahme auf das Gesamtgewicht der Kohlenwasserstofffraktion.

4. Pflanzenschutzzusammensetzung nach einem beliebigen der vorhergehenden Ansprüche, wobei die Kohlenwasserstofffraktion einen gewichtsmäßigen Gehalt an cycloalkanartigen Verbindungen im Bereich von 15 bis 45 %, noch stärker bevorzugt von 20 bis 40 % umfasst, unter Bezugnahme auf das Gesamtgewicht der Kohlenwasserstofffraktion.

5. Pflanzenschutzzusammensetzung nach einem beliebigen der vorhergehenden Ansprüche, wobei die Bestandteile der Kohlenwasserstofffraktion aus den Kohlenwasserstoffketten ausgewählt sind, welche 15 bis 30 Kohlenstoffatome, vorzugsweise 16 bis 27 Kohlenstoffatome und bevorzugt 17 bis 25 Kohlenstoffatome umfassen.

6. Pflanzenschutzzusammensetzung nach einem beliebigen der vorhergehenden Ansprüche, wobei die Kohlenwasserstofffraktion einen gewichtsmäßigen Gehalt an nicht sulfonierbaren Resten von mindestens 92 %, vorzugsweise von mindestens 95 %, bevorzugt von mindestens 99 % umfasst, unter Bezugnahme auf das Gesamtgewicht der Kohlenwasserstofffraktion.

7. Pflanzenschutzzusammensetzung nach einem beliebigen der vorhergehenden Ansprüche, wobei die Kohlenwasserstofffraktion eine Siedetemperatur im Bereich von 250 bis 400 °C, vorzugsweise von 260 bis 390 °C und bevorzugt von 270 bis 380 °C gemäß der Norm ASTM D86 hat.

8. Pflanzenschutzzusammensetzung nach einem beliebigen der Ansprüche 1 bis 7, wobei die Kohlenwasserstofffraktion bei 40 °C eine kinematische Viskosität im Bereich von 4 bis 25 mm²/s, vorzugsweise von 5 bis 20 mm²/s und stärker bevorzugt von 5 bis 10 mm²/s gemäß der Norm ASTM D445 hat.

9. Pflanzenschutzzusammensetzung nach einem beliebigen der Ansprüche 1 bis 7, wobei die Kohlenwasserstofffraktion einen Pourpoint hat, der höher oder gleich 0 °C und vorzugsweise höher oder gleich 5 °C gemäß der Norm ASTM D97 ist.

10. Pflanzenschutzzusammensetzung nach einem beliebigen der vorhergehenden Ansprüche, wobei sie in Form einer Öl-in-Wasser-Emulsion oder einer Wasser-in-ÖI-Emulsion vorliegt.

11. Emulgierbare konzentrierte Zusammensetzung, die zur Zubereitung einer Pflanzenschutzzusammensetzung gemäß einem der vorhergehenden Ansprüche verwendet werden kann, umfassend:
• mindestens eine paraffinartige Kohlenwasserstofffraktion, welche Isoparaffine, Normalparaffine und Cycloalkane umfasst, wobei der gewichtsmäßige Gehalt an Cycloalkanen im Bereich von 10 bis 50 % liegt, unter Bezugnahme auf das Gesamtgewicht der Zusammensetzung, das Gewichtsverhältnis der Normalparaffine unter Bezugnahme auf das Gewicht der Isoparaffine mindestens 0,30 beträgt, die Kohlenwasserstofffraktion einen Pourpoint hat, der höher oder gleich -5 °C gemäß der Norm ASTM D97 ist, und
• mindestens einen Emulgator.

12. Verwendung einer paraffinartigen Kohlenwasserstofffraktion, welche Isoparaffine, Normalparaffine und Cycloalkane umfasst, wobei der gewichtsmäßige Gehalt an Cycloalkanen im Bereich von 10 bis 50 % liegt, unter Bezugnahme auf das Gesamtgewicht der Zusammensetzung, das Gewichtsverhältnis der Normalparaffine unter Bezugnahme auf das Gewicht der Isoparaffine mindestens 0,30 beträgt, die Kohlenwasserstofffraktion einen Pourpoint hat, der höher oder gleich -5 °C gemäß der Norm ASTM D97 ist, zur pflanzenschützenden Behandlung von Pflanzen.

13. Verwendung nach Anspruch 12, für eine Anwendung durch Verspritzen auf angebaute Pflanzen, insbesondere durch Vernebeln oder Ausbringen.

14. Verwendung nach einem beliebigen der Ansprüche 12 bis 13, zur vorbeugenden und/oder heilenden Behandlung von Pilzerkrankungen bei Pflanzen, vorzugsweise bei Pflanzen der tropischen und äquatornahen Regionen.

15. Verwendung nach Anspruch 14, zur vorbeugenden und/oder heilenden Behandlung von Pilzerkrankungen der Bananenstaude.

16. Verwendung nach Anspruch 15, um die Entwicklung des Schwarzen Sigatoka oder der Schwarzstreifen-Krankheit der Bananenstaude, wobei diese(r) von dem Ascomyzeten-Pilz *Mycosphaerella fijiensis* verursacht wird, und/oder des gelben Sigatoka, welcher von dem Ascomyzeten-Pilz *Mycosphaerella musicola* verursacht wird, zu verringern, zu beschränken, zu verhüten, zu verhindern.

17. Verfahren zur pflanzenschützenden Behandlung von angebauten Pflanzen, das mindestens einen Schritt des Anwendens, vorzugsweise mittels des Verspritzen, Vernebeln oder Ausbringen, der Pflanzenschutzzusammensetzung nach einem beliebigen der Ansprüche 1 bis 10 umfasst.

18. Verfahren nach Anspruch 17, welche darüber hinaus die gemeinsame Anwendung mindestens eines fungiziden Wirkstoffs umfasst.

## Claims

1. Phytosanitary composition comprising at least one paraffinic hydrocarbon fraction which comprises isoparaffins, normal paraffins and naphthenes, the weight content of naphthenes ranging from 10% to 50% relative to the total weight of the composition, the weight ratio of the normal paraffins relative to the weight of the isoparaffins being greater than or equal to 0.30, the hydrocarbon fraction having a pour point of greater than or equal to -5°C according to the standard ASTM D97, in a carrier suitable for the phytosanitary treatment of plants, consisting of an oil-in-water emulsion, a water-in-oil emulsion, a dispersion or a suspension of oily particles in an aqueous phase.

2. Phytosanitary composition according to Claim 1, wherein the hydrocarbon fraction comprises a weight content of normal paraffins of greater than or equal to 15% and preferentially greater than or equal to 20%, relative to the total weight of the hydrocarbon fraction.

3. Phytosanitary composition according to either one of the preceding claims, wherein the hydrocarbon fraction comprises a weight content of isoparaffins of less than 75%, preferably less than or equal to 65% and preferentially less than or equal to 50%, relative to the total weight of the hydrocarbon fraction.

4. Phytosanitary composition according to any one of the preceding claims, wherein the hydrocarbon fraction comprises a weight content of naphthene compounds ranging from 15% to 45%, even more preferentially from 20% to 40%, relative to the total weight of the hydrocarbon fraction.

5. Phytosanitary composition according to any one of the preceding claims, wherein the components of the hydrocarbon fraction are chosen from hydrocarbon chains comprising from 15 to 30 carbon atoms, preferably from 16 to 27 carbon atoms and preferentially from 17 to 25 carbon atoms.

6. Phytosanitary composition according to any one of the preceding claims, wherein the hydrocarbon fraction comprises a weight content of unsulfonatable residues of greater than or equal to 92%, preferably greater than or equal to 95%, preferentially greater than or equal to 99%, relative to the total weight of the hydrocarbon fraction.

7. Phytosanitary composition according to any one of the preceding claims, wherein the hydrocarbon fraction has a boiling point ranging from 250 to 400°C, preferably from 260 to 390°C and preferentially from 270 to 380°C according to the standard ASTM D86.

8. Phytosanitary composition according to any one of Claims 1 to 7, wherein the hydrocarbon fraction has a kinematic viscosity at 40°C ranging from 4 to 25 mm²/s, preferentially from 5 to 20 mm²/s and more preferentially from 5 to 10 mm²/s according to the standard ASTM D445.

9. Phytosanitary composition according to any one of Claims 1 to 7, wherein the hydrocarbon fraction has a pour point of greater than or equal to 0°C and preferentially greater than or equal to 5°C according to the standard ASTM D97.

10. Phytosanitary composition according to any one of the preceding claims, which is in the form of an oil-in-water emulsion or of a water-in-oil emulsion.

11. Emulsifiable concentrated composition, that can be used for the preparation of a phytosanitary composition according to one of the preceding claims, comprising:
• at least one paraffinic hydrocarbon fraction which comprises isoparaffins, normal paraffins and naphthenes, the weight content of naphthenes ranging from 10% to 50% relative to the total weight of the composition, the weight ratio of the normal paraffins relative to the weight of the isoparaffins being greater than or equal to 0.30, the hydrocarbon fraction having a pour point of greater than or equal to -5°C according to the standard ASTM D97 and
• at least one emulsifier.

12. Use of a paraffinic hydrocarbon fraction which comprises isoparaffins, normal paraffins and naphthenes, the weight content of naphthenes ranging from 10% to 50% relative to the total weight of the composition, the weight ratio of the normal paraffins relative to the weight of the isoparaffins being greater than or equal to 0.30, the hydrocarbon fraction having a pour point of greater than or equal to -5°C according to the standard ASTM D97, for the phytosanitary treatment of plants.

13. Use according to Claim 12, for application by sprinkling on the crops, in particular by spraying or spreading.

14. Use according to either one of Claims 12 and 13, for the preventive and/or curative treatment of cryptogamic diseases of plants, preferably of plants of tropical and equatorial regions.

15. Use according to Claim 14, for the preventive and/or curative treatment of cryptogamic diseases of the banana plant.

16. Use according to Claim 15, with a view to reducing, limiting, preventing, avoiding the development of black Sigatoka disease or black leaf streak disease of the banana plant, caused by the ascomycete fungus *Mycosphaerella fijiensis* and/or of yellow Sigatoka disease caused by the ascomycete fungus *Mycosphaerella musicola.*

17. Method for the phytosanitary treatment of crops, comprising at least one step of application, preferably by sprinkling, spraying or spreading, of the phytosanitary composition according to any one of Claims 1 to 10.

18. Method according to Claim 17, which also comprises the joint application of at least one fungicidal active ingredient.
